(19) Europäisches Patentamt
European Patent Office
Office européen des brevets

(11) Veröffentlichungsnummer: **0 218 076**
**B1**

(12) # EUROPÄISCHE PATENTSCHRIFT

(45) Veröffentlichungstag der Patentschrift: **03.01.90**

(51) Int. Cl.⁴: **C07D 291/06**

(21) Anmeldenummer: **86111841.2**

(22) Anmeldetag: **27.08.86**

(54) **Verfahren zur Herstellung der nicht-toxischen Salze des 6-Methyl-3,4-dihydro-1,2,3-oxathiazin-4-on-2,2-dioxids.**

(30) Priorität: **03.09.85 DE 3531358**

(43) Veröffentlichungstag der Anmeldung: **15.04.87 Patentblatt 87/16**

(45) Bekanntmachung des Hinweises auf die Patenterteilung: **03.01.90 Patentblatt 90/1**

(84) Benannte Vertragsstaaten: **AT BE CH DE FR GB IT LI NL SE**

(56) Entgegenhaltungen:
**EP-A- 0 155 634**
**EP-A- 0 159 516**
**DE-A- 2 327 804**
**DE-A- 2 434 548**

**ANGEWANDTE CHEMIE, Band 85, Nr. 22. 1973, Seiten 965-973; K. CLAUSS et al.: "Oxathiazinondioxide, eine neue Gruppe von Süssstoffen"**

(73) Patentinhaber: **HOECHST AKTIENGESELLSCHAFT, Postfach 80 03 20, D-6230 Frankfurt am Main 80(DE)**

(72) Erfinder: **Reuschling, Dieter, Dr., Beethovenstrasse 27, D-6308 Butzbach(DE)**
Erfinder: **Linkies, Adolf, Dr., Loreleistrasse 12, D-6230 Frankfurt am Main 80(DE)**
Erfinder: **Reimann, Walter, Dr., Thüringer Weg 33, D-6238 Hofheim am Taunus(DE)**
Erfinder: **Schweikert, Otto Ernst, Dr., Freiherr-vom-Stein-Strasse 37, D-6233 Kelkheim (Taunus)(DE)**
Erfinder: **Mack, Karl Ernst, Dr., Klingenbachstrasse 43, D-6200 Wiesbaden(DE)**
Erfinder: **Ebertz, Wolfgang, Dr., Schillerring 30, D-6234 Hattersheim am Main(DE)**

**EP 0 218 076 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

6-Methyl-3,4-dihydro-1,2,3-oxathiazin-4-on-2,2-dioxid ist die Verbindung der Formel

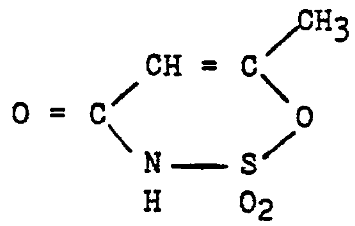

Infolge des aciden Wasserstoffs am Stickstoffatom ist die Verbindung zur Salzbildung (mit Basen) befähigt. Die nicht-toxischen Salze - wie z.B. das Na-, das K- und das Ca-Salz - können wegen ihres z.T. intensiven Süßgeschmacks als Süßstoffe auf dem Nahrungsmittelsektor verwendet werden, wobei das K-Salz ("Acesulfam K" oder auch nur "Acesulfam") von besonderer Bedeutung ist.

Zur Herstellung des 6-Methyl-3,4-dihydro-1,2,3-oxathiazin-4-on-2,2-dioxids und dessen nicht-toxischer Salze ist eine Reihe verschiedener Verfahren bekannt; vgl. Angewandte Chemie 85, Heft 22 (1973) S. 965 bis 73, entsprechend International Edition Vol. 12, No. 11 (1973), S. 869-76. Praktisch alle Verfahren gehen von Chlor- oder Fluor-sulfonylisocyanat ($XSO_2NCO$ mit X = Cl oder F) aus. Das Chlor- bzw. Fluor-sulfonylisocyanat wird dann mit Monomethylacetylen, Aceton, Acetessigsäure, Acetessigsäure-tert.-butylester oder Benzylpropenylether (in einer meist mehrstufigen Reaktion) zu Acetoacetamid-N-sulfochlorid bzw. -fluorid umgesetzt, was unter der Einwirkung von Basen (wie z.B. methanolischer KOH) cyclisiert und die entsprechenden Salze des 6-Methyl-3,4-dihydro-1,2,3-oxa-thiazin-4-on-2,2-dioxids liefert. Aus den Salzen kann das freie Oxathiazinon gewünschtenfalls auf übliche Weise (mit Säuren) erhalten werden.

Ein weiteres Verfahren zur Herstellung der Oxathiazinon-Zwischenstufe Acetoacetamid-N-sulfofluorid geht aus von Amidosulfofluorid $H_2NSO_2F$, dem partiellen Hydrolyseprodukt des Fluorsulfonylisocyanats (DE-OS 2 453 063). Danach wird das Fluorid der Amidosulfonsäure $H_2NSO_2F$ mit einer etwa äquimolaren Menge des Acetoacetylierungsmittels Diketen in einem inerten organischen Lösungsmittel in Gegenwart eines Amins bei Temperaturen zwischen etwa -30 und 100°C umgesetzt; die Umsetzung verläuft nach folgender Reaktionsgleichung (mit Triethylamin als Amin):

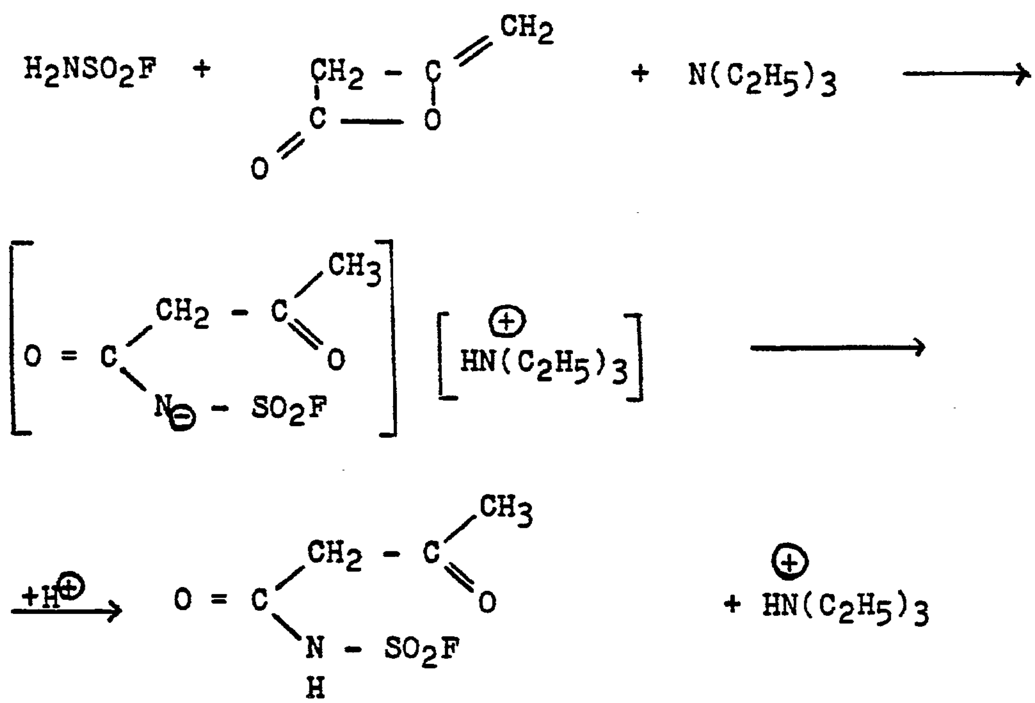

Acetoacetamid-N-sulfofluorid

Das Acetoacetamid-N-sulfofluorid wird dann auf übliche Weise mittels einer Base, z.B. mit methanolischer KOH, zum Süßstoff cyclisiert:

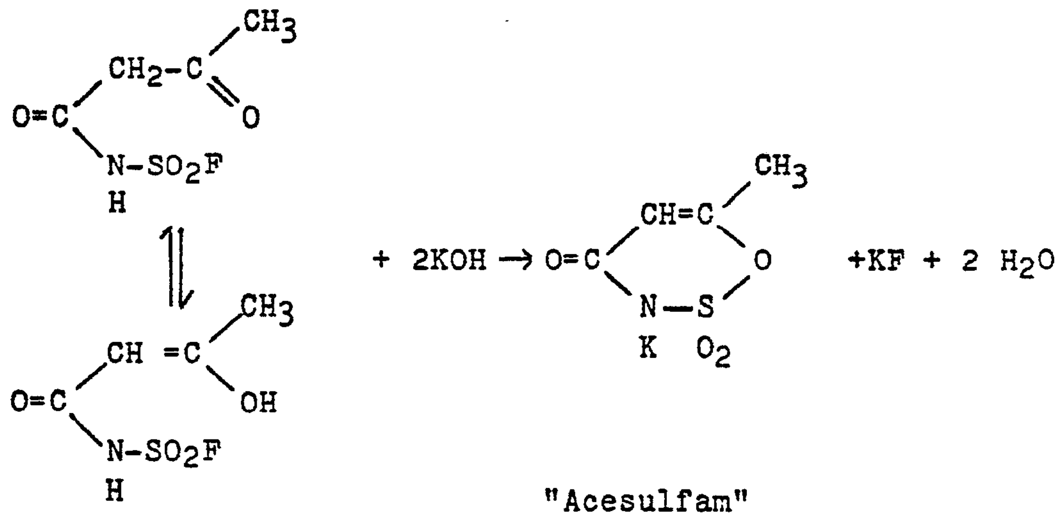

"Acesulfam"

Obwohl die bekannten Verfahren z.T. recht befriedigende Ausbeuten an 6-Methyl-3,4-dihydro-1,2,3-oxathiazin-4-on-2,2-dioxid und dessen nicht-toxischen Salzen liefern, (bis zu ca. 85 % d.Th., bezogen auf das Ausgangs-Amidosulfonsäurehalogenid), sind sie wegen der Notwendigkeit des Einsatzes der nicht ganz einfach zugänglichen Ausgangsstoffe Chlor- bzw. Fluorsulfonylisocyanat vor allem für technische Belange noch verbesserungsbedürftig; die Herstellung des Chlor- und Fluor-sulfonylisocyanats erfordert nämlich wegen der z.T. ziemlich unangenehm handzuhabenden Ausgangsmaterialien (HCN, $Cl_2$, $SO_3$ und HF) erhebliche Vorsichtsmaßnahmen und Sicherheitsvorkehrungen. Der Herstellung des Chlor- und Fluor-sulfonylisocyanats liegen folgende Reaktionsgleichungen zugrunde:

$$HCN + Cl_2 \longrightarrow ClCN + HCl$$

$$ClCN + SO_3 \longrightarrow ClSO_2NCO$$

$$ClSO_2NCO + HF \longrightarrow FSO_2NCO + HCl$$

Der Ersatz des Amidosulfofluorids in dem Verfahren gemäß der vorerwähnten DE-OS 24 53 063 etwa durch die wesentlich leichter (z.B. aus $NH_3$ + $SO_3$) erhältliche Amidosulfonsäure $H_2NSO_3H$ bzw. deren Salze erschien kaum erfolgversprechend, weil nämlich die Umsetzung des Na-Amidosulfonats $H_2NSO_3Na$ mit Diketen in wässrig-alkalischer Lösung überhaupt kein rein isolierbares Umsetzungsprodukt ergibt. Das bei dieser Umsetzung wohl zumindest mit entstandene 1:1-Addukt konnte vielmehr nur in Form des Kupplungsproduktes mit 4-Nitrophenyldiazoniumchlorid als blaßgelber Farbstoff gewonnen werden; vgl. Ber. 83 (1950), S. 551-558, insbesondere S. 555, letzter Absatz vor der Beschreibung der Versuche und S. 558, letzter Absatz:

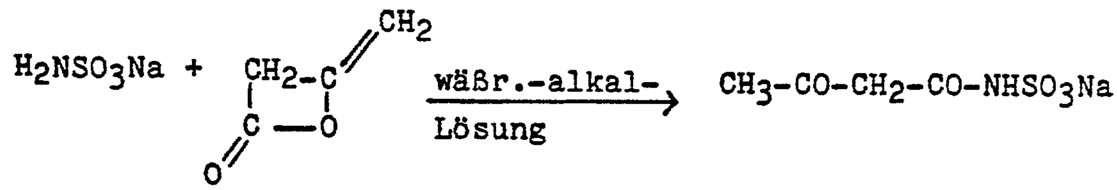


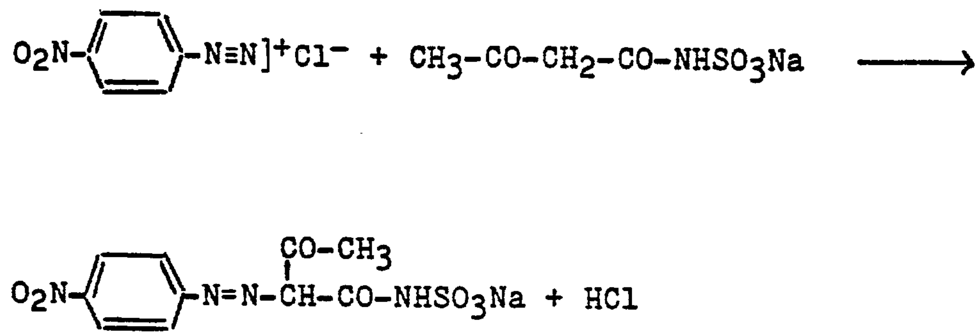

Die Acetoacetamid-N-sulfonsäure ist im übrigen ansonsten nur bzw. auch als Zwischenprodukt bei der Zersetzung des 6-Methyl-3,4-dihydro-1,2,3-oxathiazin-4-on-2,2-dioxids während des Kochens in wässriger Lösung postuliert worden; vgl. die anfangs zitierte Literatur Angew. Chemie (1973) a.a.O.:

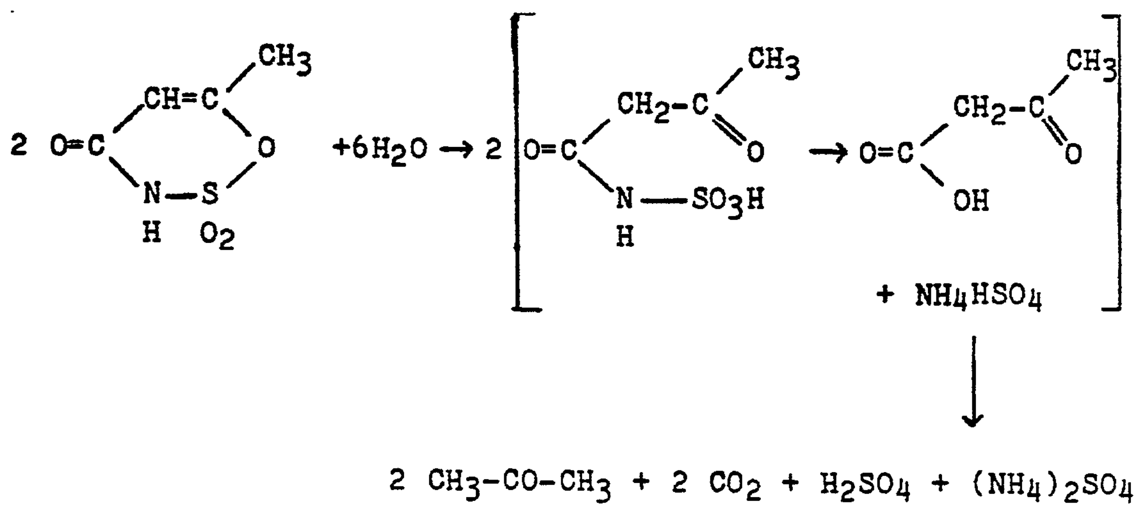

Wegen der insbesondere infolge der Notwendigkeit des Einsatzes nicht ganz einfach zugänglicher Ausgangsstoffe vor allem für die Durchführung in technischem Maßstab nicht ganz befriedigenden Verfahren des Standes der Technik zur Herstellung von 6-Methyl-3,4-dihydro-1,2,3-oxathiazin-4-on-2,2-dioxid und dessen nicht-toxischer Salze bestand somit die Aufgabe, die bekannten Verfahren entsprechend zu verbessern oder ein neues verbessertes Verfahren zu entwickeln.

Zur Lösung dieser Aufgabe wurde bereits vorgeschlagen das Verfahrens gemäß DE-OS 2 453 063 hauptsächlich in der Weise zu modifizieren, daß man das Amidosulfofluorid in dem bekannten Verfahren durch Salze der Amidosulfonsäure ersetzt und das erhaltene Acetoacetylierungsprodukt nachfolgend mittels $SO_3$ zum Ring schließt (EP-Patentanmeldung Nr. 85 102 885.2 - Veröffentlichungsnummer 0155634 - mit Priorität der deutschen Anmeldung P 34 10 439.9 vom 22.3.1984 - HOE 84/F 064).

Die zuletzt genannte Patentanmeldung bezieht sich speziell auf ein Verfahren zur Herstellung von 6-Methyl-3,4-dihydro-1,2,3-oxathiazin-4-on-2,2-dioxid und dessen nicht-toxischen Salzen durch

a) Umsetzung eines Amidosulfonsäurederivates mit einer mindestens etwa äquimolaren Menge eines Acetoacetylierungsmittels in einem inerten organischen Lösungsmittel, ggf. in Gegenwart eines Amin- oder Phosphin-Katalysators, zu einem Acetoacetamidderivat und

b) Ringschluß des Acetoacetamidderivats;

das Verfahren ist dadurch gekennzeichnet, daß man in Stufe a) als Amidosulfonsäurederivat ein in dem eingesetzten inerten organischen Lösungsmittel zumindest teilweise lösliches Salz der Amidosulfonsäure verwendet, daß man das in dieser Stufe gebildete Acetoacetamid-N-sulfonat oder auch die freie Acetoacetamid-N-sulfonsäure in Stufe b) durch die Einwirkung der mindestens etwa äquimolaren Menge von $SO_3$, gegebenenfalls in einem inerten anorganischen oder organischen Lösungsmittel, zum Ring des 6-Methyl-3,4-dihydro-1,2,3-oxathiazin-4-on-2,2-dioxids schließt und daß man das hier in der Säureform anfallende Produkt dann gegebenenfalls noch in einer Stufe c) mit einer Base neutralisiert.

Als dem Verfahren zugrundeliegenden Reaktionsgleichungen sind in der vorerwähnten Patentanmeldung angegeben (mit Diketen als Acetoacetylierungsmittel)

a)

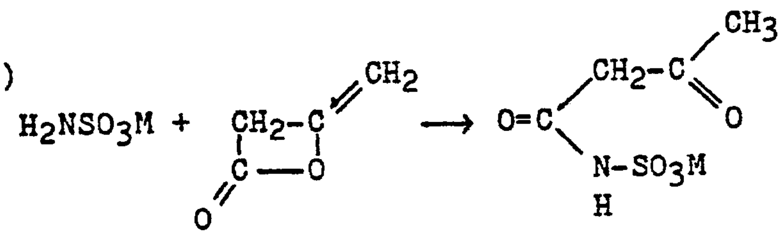

(M =Basenkation)

b)

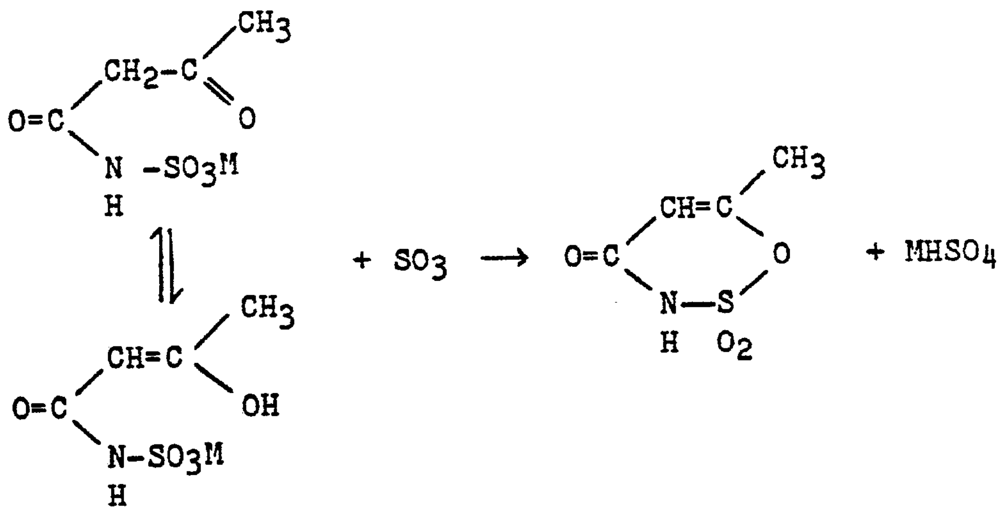

c)

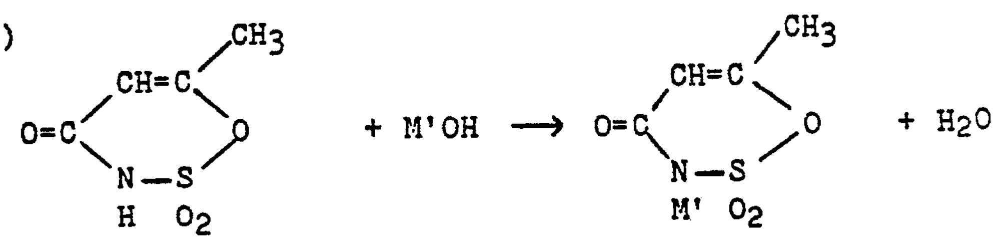

(M' = Basenkation)

In diesem Reaktionsschema ist Stufe b) mit einer gegenüber dem Acetoacetamid-N-Sulfonat äquimolaren $SO_3$-Menge dargestellt. Bevorzugt wird jedoch ein $SO_3$-Überschuß verwendet. Dabei entsteht ein in seiner chemischen Struktur noch nicht genau bekanntes Zwischenprodukt, das jedoch möglicherweise ein $SO_3$-Addukt des 6-Methyl-3,4-dihydro-1,2,3-oxathiazin-4-on-2,2-dioxids - nachfolgend als "$SO_3$-Addukt" bezeichnet - darstellt, welches dann noch hydrolysiert werden muß. In diesem Falle besteht die vorerwähnte Reaktionsstufe b) also aus 2 Teilstufen, nämlich

**b1: Ringschluß**

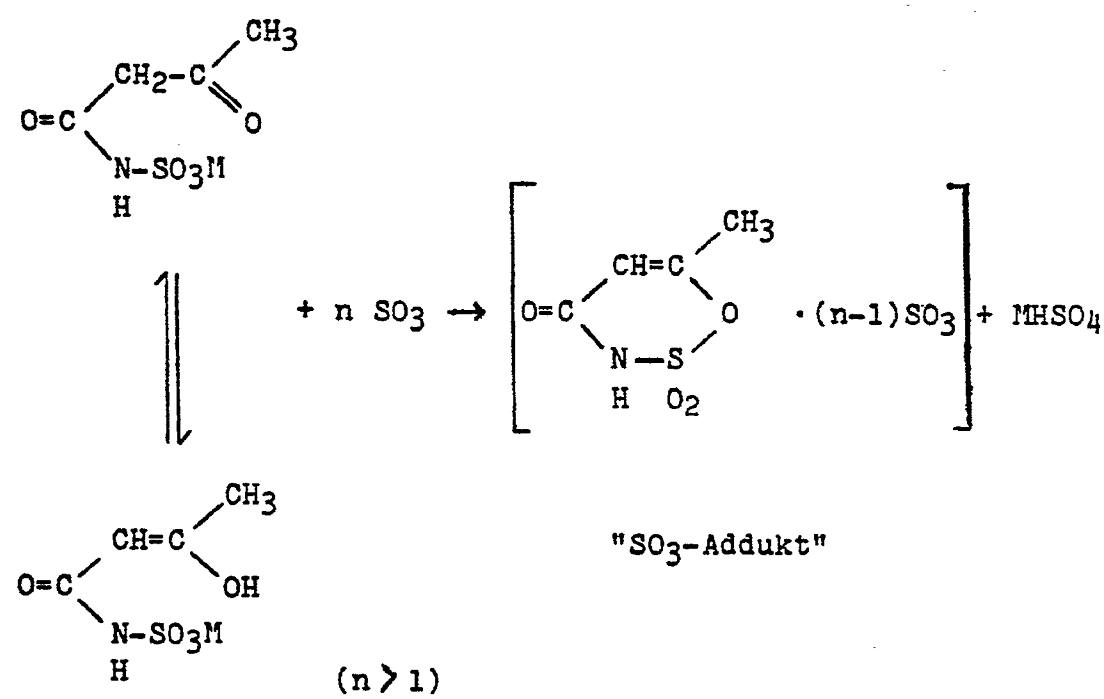


**b2: Hydrolyse**

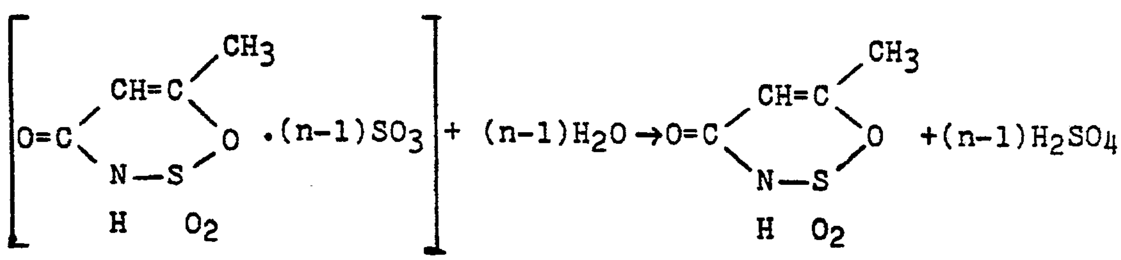

Die Ringschlußreaktion (b1) wird nach der vorerwähnten Patentanmeldung bei Temperaturen zwischen etwa -70 und 175°C, vorzugsweise zwischen –40 und +10°C, durchgeführt; die Reaktionszeiten liegen zwischen 1 und 10 Stunden.

Die Hydrolyse (b2) erfolgt nach der Ringschlußreaktion durch Zugabe von Wasser oder Eis.

Die Aufarbeitung geschieht dann auf übliche Weise, näher wird die Aufarbeitung jedoch nur für den bevorzugten Fall der Verwendung von Methylenchlorid als Reaktionsmedium erläutert. In diesem Fall bilden sich nach der Hydrolyse 2 Phasen, wobei sich das 6-Methyl-3,4-dihydro-1,2,3-oxathiazin-4-on-2,2-dioxid hauptsächlich in die organische Phase begibt. Die noch in der wäßrigen Schwefelsäure befindlichen Anteile können durch Extraktion mit einem (mit Wasser nicht mischbaren) organischen Lösungsmittel wie z.B. mit Methylenchlorid oder einem organischen Ester gewonnen werden.

Oder man destilliert nach der Zugabe von Wasser das Reaktionslösemittel ab und extrahiert das in der Reaktionsschwefelsäure zurückbleibende 6-Methyl-3,4-dihydro-1,2,3-oxathiazin-4-on-2,2-dioxid mit einem besser geeigneten organischen Lösemittel.

Die vereinigten organischen Phasen werden z.B. mit $Na_2SO_4$ getrocknet und eingeengt. Bei der Extraktion gegebenenfalls mitgerissene Schwefelsäure kann man durch gezielte Zugabe wäßriger Lauge zur organischen Phase entfernen. Falls die Gewinnung des 6-Methyl-3,4-dihydro-1,2,3-oxathiazin-4-on-2,2-dioxids beabsichtigt ist, wird es zweckmäßig noch auf übliche Weise gereinigt (vorzugsweise durch Umkristallisation). Die Ausbeute liegt zwischen 70 und 95% d.Th., bezogen auf das Acetoacetamid-N-sulfonat (bzw. die freie Säure).

Wenn jedoch die Gewinnung eines nicht-toxischen Salzes des 6-Methyl-3,4-dihydro-1,2,3-oxathiazin-4-on-2,2-dioxids beabsichtigt ist, folgt noch die Neutralisationsstufe c). Dazu wird die in Stufe b) in der Säureform anfallende Oxathiazinon-Verbindung auf übliche Weise mit einer entsprechenden Base neutralisiert. Zu diesem Zweck werden beispielsweise die am Ende der Stufe b) vereinigten, getrockneten und eingeengten organischen Phasen in geeigneten organischen Lösungsmitteln, wie z.B. Alkoholen, Ketonen, Estern oder Ethern oder auch in Wasser mit einer entsprechenden Base – vorzugsweise mit einer Kaliumbase wie z.B. KOH, $KHCO_3$, $K_2CO_3$, K-Alkoholate etc., neutralisiert. Oder die Oxathiazinon-Verbindung wird aus der gereinigten organischen Extraktionsphase (Stufe b) mit wässriger Kaliumbase direkt extraktiv neutralisiert. Das Oxathiazinon-Salz fällt dann, gegebenenfalls nach Einengen der Lösung, in kristalliner Form aus und kann zur Reinigung noch umkristallisiert werden. Die Neutralisationsstufe verläuft mit praktisch 100%iger Ausbeute.

Hinsichtlich der weiteren Verfahrensdetails wird auf die ausführliche Beschreibung in der genannten Patentanmeldung verwiesen.

Das Verfahren geht von einfach zugänglichen und wohlfeilen Ausgangsstoffen aus und ist außerordentlich einfach durchführbar. Die Ausbeuten des Gesamtverfahrens liegen zwischen 65 und 95% d.Th., bezogen auf das Ausgangs-Amidosulfonat.

Im Zuge der weiteren Bearbeitung dieses Verfahrens wurde auch vorgeschlagen, sowohl die Ringschlußreaktion (b1) als auch die Hydrolyse (b2) in kurzen bis sehr kurzen Zeiten (ca. 10 Minuten bis herab in den Bereich von Sekunden und Sekundenbruchteilen) durchzuführen (Patentanmeldung P 3 527 070.5 vom 29. 7. 1985 – DE-A 3 527 070. Die praktische Ausführung geschieht vorzugsweise in Vorrichtungen, welche für die Durchführung derartiger schnell unter Wärmeentwicklung ablaufender Reaktionen geeignet und bekannt sind (Dünnschicht-, Fallfilm-, Sprühreaktoren, Rohrreaktoren mit und ohne Einbauten, etc.). Die Aufarbeitung des Reaktionsgemisches erfolgt wie in der zuvor genannten Patentanmeldung beschrieben. Durch diese «Kurzzeitvariante» läßt sich die technische Durchführung und insbesondere die Raum-Zeit-Ausbeute des Verfahrens erheblich verbessern.

Schließlich wurde auch schon vorgeschlagen, anstelle der Stufen a) und b) des Verfahrens der vorerwähnten EP-A 155 634, Anm.-Nr. 85 102 885.2 Acetoacetamid mit der mindestens etwa 2-molaren Menge $SO_3$, gegebenenfalls in einem inerten anorganischen oder organischen Lösungsmittel, umzusetzen (DE-Patentanmeldung P 3 410 440.2 vom 22. 3. 1984 – DE-A 3 410 440).

Hierbei entsteht in einem Schritt wahrscheinlich zuerst aus einem Mol Acetoacetamid und einem Mol $SO_3$ Acetoacetamid-N-sulfonsäure, die dann mit einem weiteren Mol $SO_3$ zum 6-Methyl-3,4-dihydro-1,2,3-oxathiazin-4-on-2,2-dioxid cyclisiert entsprechend dem folgenden Reaktionsschema:

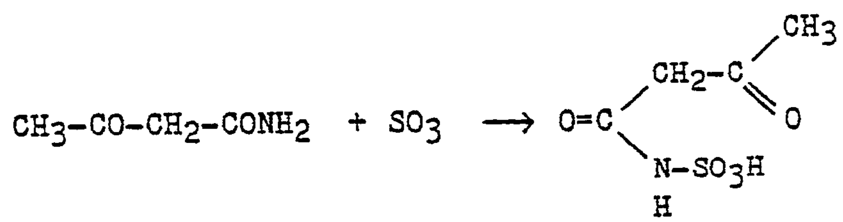

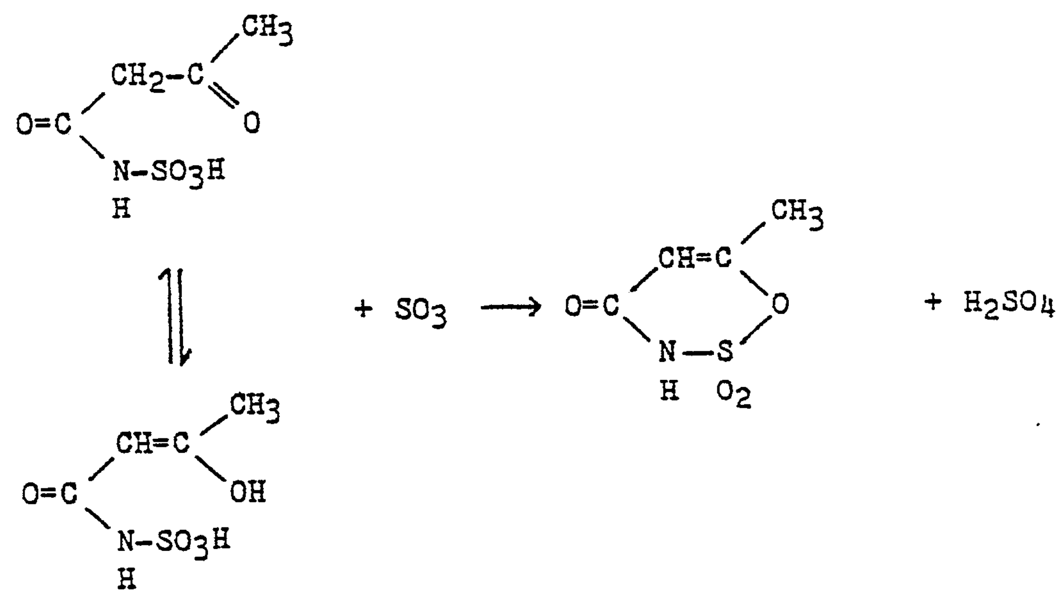

Mit überschüssigem $SO_3$ entsteht auch hier das «$SO_3$-Addukt», das zwecks Freisetzung des 6-Methyl-3,4-dihydro-1,2,3-oxathiazin-4-on-2,2-dioxids noch hydrolysiert werden muß. Die Aufarbeitung des hydrolysierten Ansatzes sowie gegebenenfalls die Überführung des 6-Methyl-3,4-dihydro-1,2,3-oxathiazin-4-on-2,2-dioxids in dessen nicht-toxische Salze geschieht im Prinzip in der gleichen Weise wie dies in der vorerwähnten EP-A 155 634, Anm.-Nr. 85 102 885.2 beschrieben ist. Die Ausbeuteangaben für das 6-Methyl-3,4-dihydro-1,2,3-oxathiazin-4-on-2,2-dioxid liegen zwischen 30 und 90% d.Th., bezogen auf das Ausgangs-Acetoacetamid.

Nach allen drei vorerwähnten Patentanmeldungen wird das bei der Hydrolyse des «$SO_3$-Addukts» in Freiheit gesetzte 6-Methyl-3,4-dihydro-1,2,3-oxathiazin-4-on-2,2-dioxid aus der organischen Phase gewonnen, welche sich bei Verwendung eines (mit Wasser nicht mischbaren) organischen Reaktionslösemittels nach dem Wasserzusatz bildet und/oder welche bei der Extraktion der Reaktionsschwefelsäure mit organischen Lösemitteln entsteht. Das so gewonnene 6-Methyl-3,4-dihydro-1,2,3-oxathiazin-4-on-2,2-dioxid sowie auch die daraus gegebenenfalls durch Umsetzung mit entsprechenden Basen erhaltenen nicht-toxischen Salze sind jedoch nicht immer von der erforderlichen Reinheit, so daß oft noch verschiedene – mit zusätzlichem Aufwand sowie auch mit Substanzverlusten verbundene – Reinigungsoperationen – vorzugsweise Umkristallisation(en) – notwendig sind.

Die in der EP-A 155 634, Anm.-Nr. 85 102 885.2 erwähnte Möglichkeit der Gewinnung der Oxathiazinon-Verbindung aus der gereinigten organischen Extraktionsphase durch extraktive Neutralisation mit einer wäßrigen Kaliumbase ist dort insbesondere in Beispiel 11 näher erläutert. Dort wird für die Reinigung der organischen Extraktionsphase zwecks Neutralisation von mitgerissener Schwefelsäure wäßrige Lauge verwendet.

In weiterer Ausbildung der vorerwähnten Verfahren wurde nun gefunden, daß man durchweg reinere Salze des 6-Methyl-3,4-dihydro-1,2,3-oxathiazin-4-on-2,2-dioxids erhält, wenn man die – wie vorstehend beschrieben erhaltene – organische Phase vor ihrer weiteren Aufarbeitung durch Extraktion mit einem geringeren Volumen Wasser oder verdünnter wäßriger Schwefelsäure, wobei Wasser bevorzugt ist, reinigt.

Erfindungsgegenstand ist daher ein Verfahren zur Herstellung der nicht-toxischen Salze des 6-Methyl-3,4-dihydro-1,2,3-oxathiazin-4-on-2,2-dioxids durch Ringschluß eines Acetoacetamid-Derivates und Neutralisation mit Basen; das Verfahren ist dadurch gekennzeichnet,

– daß man als Acetoacetamid-Derivat Acetoacetamid-N-sulfonsäure oder deren Salze – gelöst in einem mit Wasser nicht mischbaren inerten organischen Lösemittel – verwendet,

– daß man den Ringschluß durch die Einwirkung der mindestens äquimolaren Menge $SO_3$ – gegebenenfalls in gleicher Weise gelöst in einem mit Wasser nicht mischbaren inerten organischen Lösemittel oder auch in einem inerten anorganischen Lösemittel – durchführt,

– daß man im Falle des Einsatzes einer mehr als äquimolaren Menge $SO_3$ das nach der Ringschlußreaktion als $SO_3$-Addukt anfallende 6-Methyl-3,4-dihydro-1,2,3-oxathiazin-4-on-2,2-dioxid hydrolysiert,

– daß man die vorliegende oder bei der Hydrolyse sich absondernde organische Phase durch Extraktion mit Wasser oder 2 bis 20%iger wäßriger Schwefelsäure – vorzugsweise nur mit Wasser – im Volumenverhältnis der organischen Phase zur wäßrigen bzw. wäßrig-schwefelsauren Phase von (20–5):1 reinigt

– und daß man aus der so gereinigten organischen Phase die nicht-toxischen Salze des 6-Methyl-3,4-dihydro-1,2,3-oxathiazin-4-on-2,2-dioxids durch Neutralisation mit Basen gewinnt.

Das glatte Gelingen des Ringschlusses der Acetoacetamid-N-sulfonsäure und von deren Salzen mit $SO_3$ ist sehr überraschend, weil die unter Ringschluß erfolgende Wasser- bzw. Basen-Abspaltung nämlich mit anderen Wasser- bzw. Basen-abspaltenden Mitteln wie z.B. $P_2O_5$, Acetanhydrid, Trifluoressigsäureanhydrid, Thionylchlorid etc. nicht oder jedenfalls praktisch nicht gelingt, wie bereits in der vorerwähnten EP-Patentanmeldung Nr. 85 102 885.2 (EP-A 155 634) anhand eines Vergleichsbeispiels (mit $P_2O_5$) gezeigt werden konnte.

Außerdem ist überraschend, daß die nach dem Verfahren erhaltenen Salze des 6-Methyl-3,4-dihydro-1,2,3-oxathiazin-4-on-2,2-dioxids in außerordentlich reiner Form (Reinheitsgrad durchweg über 99%) anfallen, weil durchaus nicht ohne weiteres zu erwarten war, daß durch eine einfache Extraktion der organischen Phase mit Wasser oder mit verdünnter wäßriger Schwefelsäure praktisch alle störenden Verunreinigungen entfernt werden.

Die Herstellung der Ausgangs-Acetoacetamid-N-sulfonsäure und von deren Salzen erfolgt bevorzugt nach Stufe a) des Verfahrens der vorerwähnten EP-Patentanmeldung Nr. 85 102 885.2 (EP-A 155 634) durch Umsetzung der Li- oder Ammonium-Salze der Amidosulfonsäure mit Diketen in inerten organischen Lösungsmitteln. Hierbei werden Lösungen der Li- und Ammonium-Salze der Acetoacetamid-N-sulfonsäure erhalten, welche als solche direkt für die Ringschlußreaktion mit $SO_3$ eingesetzt werden können.

Für die genannte Ringschlußreaktion können natürlich auch andere Salze der Acetoacetamid-N-sulfonsäure – insbesondere Alkali- und Erdalkalisalze – verwendet werden. Der Einsatz der freien Acetoacetamid-N-sulfonsäure bringt gegenüber den Salzen kaum Vorteile.

Wie die Salze, so kann auch die freie Acetoacetamid-N-sulfonsäure gleich in der entsprechenden Lösung wie sie bei der Herstellung anfällt, für die Ringschlußreaktion eingesetzt werden. Als Lösung wie sie bei der Herstellung anfällt, kann auch die Lösung der beim Verfahren der DE-Patentanmeldung P 3 410 440.2 (DE-A 3 410 440) wohl intermediär gebildeten freien Acetoacetamid-N-sulfonsäure betrachtet werden.

Als inerte organische Lösemittel für die Acetoacetamid-N-sulfonsäure oder für deren Salze kommen zweckmäßig aus der Reihe der in den vorerwähnten Patentanmeldungen aufgeführten inerten organischen Lösemittel diejenigen in Frage, welche mit Wasser nicht mischbar sind; d.s.
halogenierte aliphatische Kohlenwasserstoffe, vorzugsweise mit bis zu 4 C-Atomen wie z.B. Methylenchlorid, Chloroform, 1,2-Dichlorethan, Trichlorethylen, Tetrachlorethylen, Trichlor-fluorethylen etc.;
Kohlensäureester mit niederen aliphatischen Alkoholen, vorzugsweise mit Methanol oder Ethanol;
Nitroalkane, vorzugsweise mit bis zu 4 C-Atomen, insbesondere Nitromethan; und
alkylsubstituierte Pyridine, vorzugsweise Collidin, etc.

Die organischen Lösemittel können sowohl einzeln als auch in Mischung eingesetzt werden.

Besonders bevorzugte Lösemittel sind halogenierte aliphatische Kohlenwasserstoffe, insbesondere Methylenchlorid.

Die Konzentration der Acetoacetamid-N-sulfonsäure oder von deren Salzen im inerten Lösemittel ist nicht kritisch, wird aber einerseits begrenzt durch die Löslichkeit, andererseits durch Wirtschaftlichkeitsüberlegungen, da bei hoher Verdünnung viel Lösemittel nachher wieder abgetrennt und aufgearbeitet werden muß. Im allgemeinen sind Konzentrationen zwischen 0,1 und 2 Mol Acetoacetamid-N-sulfonsäure oder von deren Salzen pro Liter zweckmäßig.

Das $SO_3$ kann sowohl in fester oder flüssiger Form als auch durch Einkondensation von $SO_3$-Dampf zugegeben werden. Bevorzugt ist jedoch der Zusatz in gelöster Form, und zwar gelöst in einem mit Wasser nicht mischbaren inerten organischen Lösemittel oder auch in einem inerten anorganischen Lösemittel.

Als mit Wasser nicht mischbare inerte organische Lösemittel kommen im Prinzip die gleichen in Frage, welche auch für die Lösung der Acetoacetamid-N-sulfonsäure oder von deren Salzen verwendet werden.

Als inerte anorganische Lösemittel können beispielsweise konzentrierte Schwefelsäure oder flüssiges $SO_2$ eingesetzt werden. Auch die Menge des für das $SO_3$ eingesetzten inerten Lösemittels ist im Prinzip nicht kritisch. Wenn ein Lösemittel eingesetzt wird, soll lediglich eine ausreichende Lösung des $SO_3$ gewährleistet sein; nach oben ist die Menge des Lösemittels von Wirtschaftlichkeitserwägungen begrenzt. Günstige Konzentrationen liegen bei 5 bis 50 Gew.-%, vorzugsweise 15 bis 30 Gew.-% $SO_3$.

In einer bevorzugten Ausführungsform der Erfindung wird sowohl für die Acetoacetamid-N-sulfonsäure bzw. deren Salze als auch für das $SO_3$ das gleiche inerte Lösemittel, vorzugsweise aus der Gruppe der halogenierten aliphatischen Kohlenwasserstoffe, insbesondere nur Methylenchlorid, verwendet.

Das Molverhältnis von Acetoacetamid-N-sulfonsäure bzw. -sulfonat: $SO_3$ kann zwar 1:1 sein, bevorzugt ist aber ein bis zu 20facher $SO_3$-Überschuß, vorzugsweise ein etwa 3- bis 10facher, insbesondere 4- bis 7facher molarer Überschuß.

Die Durchführung der Ringschlußreaktion erfolgt ansonsten im Prinzip in der gleichen Weise und unter den gleichen Bedingungen wie dies in den vorerwähnten drei Patentanmeldungen beschrieben ist.

Wenn die Acetoacetamid-N-sulfonsäure oder deren Salze und das $SO_3$ in äquimolarer Menge eingesetzt werden, entsteht – wie aus den anfangs wiedergegebenen Reaktionsschemata ersichtlich ist – kein «$SO_3$-Addukt». Eine Hydrolyse ist daher in diesem Fall nicht erforderlich.

Im Falle des Einsatzes der Ausgangsverbindungen – gelöst in inerten organischen Lösungsmitteln – stellt der Reaktionsansatz dann die organische Phase dar, welche ohne weitere Trennoperationen oder gegebenenfalls nach Abtrennung ausgefallener Salze, gleich der erfindungsgemäßen Weiterverarbeitung zugeführt werden kann.

Wenn das Ausgangs-$SO_3$ gelöst in einem inerten anorganischen Lösemittel wie z.B. in konz. Schwefelsäure eingesetzt wird, muß nach beendeter Ringschlußreaktion die organische Phase entsprechend abgetrennt werden.

Im bevorzugten Fall des Einsatzes von Acetoacetamid-N-sulfonsäure oder von deren Salzen und $SO_3$ im Molverhältnis 1: mehr als 1 entsteht bei der Ringschlußreaktion ein «$SO_3$-Addukt», aus dem das 6-Methyl-3,4-dihydro-1,2,3-oxathiazin-4-on-2,2-dioxid durch Hydrolyse freigesetzt werden muß. Die Hydrolyse erfolgt durch Zugabe von Wasser oder Eis, zweckmäßig in einer – im Verhältnis zum angewandten $SO_3$-Überschuß – 2- bis 6fachen molaren Menge.

Nach der Hydrolyse liegt dann ein 2- oder (wenn bereits 6-Methyl-3,4-dihydro-1,2,3-oxathiazin-4-on-2,2-dioxid ausgefallen ist) 3-Phasengemisch vor. Das 6-Methyl-3,4-dihydro-1,2,3-oxathiazin-4-on-2,2-dioxid befindet sich im wesentlichen gelöst in der organischen und in der Schwefelsäurephase. Die organische Phase wird dann abgetrennt.

Vorzugsweise wird die wäßrig-schwefelsaure Phase – zusammen mit gegebenenfalls ausgefallenem 6-Methyl-3,4-dihydro-1,2,3-oxathiazin-4-on-2,2-dioxid – noch mit einem mit Wasser nicht mischbaren inerten organischen Lösungsmittel – insbesondere dem gleichen, in dem auch die Ringschlußreaktion durchgeführt wurde – extrahiert und mit der vorher abgetrennten organischen Phase vereinigt.

Falls das für die Ringschlußreaktion verwendete inerte organische Lösungsmitel etwa durch Verdampfung gemäß der «Kurzzeitvariante» nach DE-A 3 527 070, Anm.-Nr. P 3 727 070.5 bereits entfernt ist, befindet sich das 6-Methyl-3,4-dihydro-1,2,3-oxathiazin-4-on-2,2-dioxid hauptsächlich gelöst nur in der Schwefelsäurephase. In diesem Fall müßte das 6-Methyl-3,4-dihydro-1,2,3-oxathiazin-4-on-2,2-dioxid mit einem inerten organischen Lösungsmittel der vorher beschriebenen Art für die erfindungsgemäße Aufarbeitung wieder möglichst vollständig extrahiert werden.

Die von der wäßrig-schwefelsauren Phase abgetrennte organische Phase bzw. die entsprechenden vereinigten organischen Phasen werden sodann durch Extraktion mit einem geringeren Volumen Wasser oder verdünnter wäßriger Schwefelsäure gereinigt; bevorzugt ist die Reinigung nur mit Wasser. Wenn für die Reinigung verdünnte wäßrige Schwefelsäure verwendet wird, ist solche einer Konzentration zwischen 2 und 20% bevorzugt.

Das Volumenverhältnis der organischen Phase zur wäßrigen bzw. wäßrig-schwefelsauren Extraktionsphase liegt im allgemeinen bei (20–5):1. Auch mit wesentlich geringeren Wassermengen kann jedoch oft noch eine effektvolle Reinigung erzielt werden.

Die Extraktion erfolgt im einfachsten Fall durch Rühren der beiden Phasen in einem Rührkolben oder einem Rührkessel; als spezielle Vorrichtungen kommen im Prinzip alle technischen Extraktionsapparate in Frage wie z.B. Mixer-Settler-Apparaturen, Siebbodenkolonnen, Füllkörperkolonnen, Karr-Kolonnen etc. Auch Mischelemente wie z.B. statische Mischer können zur Intensivierung des Kontakts der Extraktionsphasen herangezogen werden.

Die Extraktion kann sowohl diskontinuierlich als auch kontinuierlich durchgeführt werden.

Der Anteil an extrahiertem 6-Methyl-3,4-dihydro-1,2,3-oxathiazin-4-on-2,2-dioxid liegt im allgemeinen je nach der eingesetzten Wassermenge zwischen 2 und 30 (Gew.)-%. Für die Wirtschaftlichkeit des Gesamtverfahrens ist es von Bedeutung, die Wasserphase (mit den relativ geringen Mengen an extrahiertem 6-Methyl-3,4-dihydro-1,2,3-oxathiazin-4-on-2,2,-dioxid) wieder in die Hydrolyse des «$SO_3$-Addukts» zurückzuführen. Dies kann sowohl chargenweise als auch kontinuierlich erfolgen.

Aus der gereinigten organischen Phase bzw. den gereinigten vereinigten organischen Phasen werden die nicht-toxischen Salze des 6-Methyl-3,4-dihydro-1,2,3-oxathiazin-4-on-2,2-dioxids durch Neutralisation mit Basen gewonnen. Als Basen kommen hier diejenigen mit nicht-toxischen Kationen in Frage. Bevorzugt sind Kaliumbasen (Lösungen von KOH, $KHCO_3$, $K_2CO_3$ etc.), insbesondere KOH.

Die Neutralisation des 6-Methyl-3,4-dihydro-1,2,3-oxathiazin-4-on-2,2-dioxids und die Gewinnung von dessen nicht-toxischen Salzen aus der das 6-Methyl-3,4-dihydro-1,2,3-oxathiazin-4-on-2,2-dioxid enthaltenden gereinigten organischen Phase geschieht mit Vorteil z.B. durch Eindampfen der organischen Phase, Aufnahme des Rückstandes in Wasser oder einem niederen aliphatischen Alkohol, Neutralisation mit wäßriger oder wäßrig-alkoholischer Base und Kristallisation aus dieser Lösung,
oder etwa auch durch intensiven Kontakt der gereinigten organischen Phase bzw. der entsprechenden vereinigten organischen Phasen mit wäßriger Lauge. Der intensive Kontakt erfolgt im allgemeinen nach Art und Weise einer Extraktion nach den hierfür üblichen Verfahren in den üblichen Vorrichtungen, wie sie bereits voranstehend beschrieben wurden. Auch Mischelemente wie z.B. statische Mischer können hier verwendet werden.

Bei der Neutralisation wird im allgemeinen so viel Base zugesetzt, bis die wäßrig-alkoholische bzw. die nur wäßrige Phase einen pH-Wert von 5 bis 12, vorzugsweise 8 bis 11 erreicht. Aus der wäßrig-alkoholischen bzw. nur wäßrigen Phase wird das 6-Methyl-3,4-dihydro-1,2,3-oxathiazin-4-on-2,2-dioxid-Salz dann auf übliche Weise (durch Kristallisation) gewonnen.

Wenn man für die Neutralisation beim intensiven Kontakt der gereinigten organischen Phasen mit wäßrigen Basen z.B. verdünnte wäßrige KOH einer Konzentration zwischen 1 und 10%, vorzugsweise 4 bis 8%, verwendet, erfolgt die Salzgewinnung nach der Abtrennung der wäßrigen Phase durch deren Einengung und Abkühlung, wobei kristallines Acesulfam K ausfällt, das normalerweise nach dem Trocknen einen Reingehalt von über 99,5% aufweist. Der Rest (zirka 0,5%) ist Kaliumsulfat.

Wenn man die Neutralisation mit konzentrierterer wäßriger KOH – d.h. Kalilauge einer Konzentration zwischen 10 und 50%, vorzugsweise zwischen 20 und 35% – durchführt, kristallisiert ein Teil des gebildeten Acesulfam K gleich während des intensiven Kontakts der Kalilauge mit der entsprechenden gereinigten organischen Phase direkt aus. Auch dieses Produkt besitzt einen Reinheitsgrad von normalerweise über 99,5%. Durch Einengen und gegebenenfalls Abkühlung der wäßrigen Lösung wird das weitere Acesulfam K gewonnen.

Es ist von Vorteil die beiden voranstehend beschriebenen Neutralisationsstufen so durchzuführen, daß der intensive Kontakt der wäßrigen Base mit der organischen Phase sehr rasch – vorzugsweise in Zeiten von 1 sec. bis 60 sec., insbesondere von 2 bis 10 sec. – erfolgt. Dies hat eine Steigerung der Raum-Zeit-Ausbeute dieser Stufe zur Folge. Als Vorrichtungen hierfür kommen z.B. Dünnschichtreaktoren, Fallfilmreaktoren oder Mischelemente in Betracht.

Eine weitere bevorzugte Ausführungsform der Neutralisation des 6-Methyl-3,4-dihydro-1,2,3-oxathiazin-4-on-2,2-dioxids besteht darin, daß man aus der diese Verbindung enthaltenden (gereinigten) organischen Phase das organische Lösungsmittel unter gleichzeitiger Zugabe von Wasser verdampft und die dabei erhaltene wäßrige Lösung des 6-Methyl-3,4-dihydro-1,2,3-oxathiazin-4-on-2,2-dioxids mit einer Base, vorzugsweise mit einer Kaliumbase, insbesondere mit KOH, neutralisiert. Diese Ausführungsform funktioniert aber nur, wenn die entsprechende organische Phase Lösemittel mit Siedepunkten unter

100°C (bei Normaldruck) enthält, da andernfalls noch das zudosierte Wasser gleich mit verdampfen würde. Bevorzugte Vorrichtungen für diese Ausführungsform sind Geräte zur schnellen Verdampfung, wie z.B. Dünnschichtverdampfer oder Fallfilmverdampfer.

Eine weitere bevorzugte Ausführungsform der Erfindung besteht darin, daß man zur Neutralisation des 6-Methyl-3,4-dihydro-1,2,3-oxathiazin-4-on-2,2-dioxids aus der diese Verbindung enthaltenden (gereinigten) organischen Phase das organische Lösemittel unter gleichzeitiger Zugabe einer wäßrigen Base, vorzugsweise einer wäßrigen Kaliumbase, insbesondere von wäßriger KOH, verdampft. Auch diese Ausführungsform funktioniert nur, wenn die organischen Lösemittel Siedepunkte unter 100°C (bei Normaldruck) aufweisen. Bevorzugte Vorrichtungen für diese Ausführungsform sind ebenfalls Geräte zur schnellen Verdampfung, wie z.B. Dünnschichtverdampfer oder Fallfilmverdampfer. Es resultiert hier eine erhitzte wäßrige Lösung, aus der beim Abkühlen sowie gegebenenfalls Eindampfen das 6-Methyl-3,4-dihydro-1,2,3-oxathiazin-4-on-2,2-dioxid-Salz auskristallisiert.

Bei allen voranstehenden Ausführungsformen der Neutralisation können Ausbeuten (= Isolierungsgrad) von durchweg 80–90%, bezogen auf das vor der Neutralisation in der organischen Phase vorhandene 6-Methyl-3,4-dihydro-1,2,3-oxathiazin-4-on-2,2-dioxid, erreicht werden. Falls gewünscht, kann die Ausbeute durch zusätzliches Eindampfen der nach Abtrennen des Acesulfams K anfallenden wäßrigen Phasen noch gesteigert werden. Bezogen auf die Ausgangs-Acetoacetamid-N-sulfonsäure bzw. -sulfonate liegen die Ausbeuten um den Faktor der Reaktionsausbeute niedriger.

Für extreme Reinheitsforderungen kann das Acesulfam K noch zusätzlich aus Wasser umkristallisiert werden, gegebenenfalls in Gegenwart von Aktivkohlen.

Die folgenden Beispiele sollen der weiteren Erläuterung der Erfindung dienen. In den Beispielen wird 6-Methyl-3,4-dihydro-1,2,3-oxathiazin-4-on-2,2-dioxid mit «ASH», dessen Kaliumsalz mit «ASK» abgekürzt.

Herstellung des für die Beispiele verwendeten Ausgangs-Acetoacetamid-N-sulfonats

97,1 g (1,0 Mol) Amidosulfonsäure wurden in 1,0 l Methylenchlorid suspendiert. Unter Rühren wurden 106 g (1,05 Mol) Triethylamin zugefügt, wobei die Amidosulfonsäure als Triethylammoniumsalz in Lösung ging. Nach Zugabe von 6 g (0,1 Mol) Eisessig wurden unter Rühren bei einer Innentemperatur von 15°C 93,8 g (1,08 Mol) 97%iges Diketen innerhalb von 1 Stunde eingetropft. Nach HPLC (= Hochdruckflüssig-Chromatographie)-Analyse lag die Ausbeute an Acetoacetamid-N-sulfonat bei 90%. Die so erhaltene Lösung wurde direkt für die weitere Umsetzung eingesetzt.

Beispiel 1

Ringschluß und Hydrolyse

In einem Rundkolben mit Stickstoff-Überlagerung wurden 400 ml einer 15 %igen Lösung von $SO_3$ in $CH_2Cl_2$ vorgelegt. Bei einer Innentemperatur von -30°C (Isopropanol/Trockeneis-Kühlung: -40 bis -50°C) wurden unter Rühren innerhalb von 25 Min. 1850 ml der $SO_3/CH_2Cl_2$-Lösung (15 %ig) und gleichzeitig die oben beschriebene Lösung von Acetoacetamid-N-sulfonat in $CH_2Cl_2$ zugetropft.

Zur Hydrolyse wurden unter starker Außenkühlung, beginnend bei -30°C, innerhalb von ca. 30 Min. 500 ml Wasser eingetropft. Die Temperatur stieg hierbei rasch von -30°C auf 0°C an; später wurde sie bei 0 bis +5°C gehalten.

Herstellung einer ASH/Methylenchlorid-Lösung

Bei 5°C wurde die organische Phase abgetrennt und die wässrige, schwefelsaure Phase noch zweimal mit je 1,0 l $CH_2Cl_2$ extrahiert. Es wurde eine Lösung von 132 g ASH in 5,0 l Methylenchlorid (= 1,9 %ige Lösung) erhalten. Ausbeute: 81 % (bezogen auf Amidosulfonsäure).

Gewinnung von ASK

2,5 l dieser ASH/Methylenchlorid-Lösung wurden 2 Stunden lang mit 250 ml Wasser gerührt. Die organische Phase wurde dann im Vakuum eingedampft. Der Rückstand wurde in derselben Gewichtsmenge Methanol gelöst und danach mit 20 %iger KOH/Methanol auf pH 8 - 10 gebracht (= KOH/Methanol-Fällung). Nach Abfiltrieren und Trocknen wurden 69,5 g ASK isoliert (Ausbeute: 85 %, bezogen auf 66 g eingesetztes ASH).

| Analyse: | ASK | $K_2SO_4$ |
|---|---|---|
| | 99,6% | 0,4% |

Vergleich:

2,5 l der wie vorstehend beschrieben hergestellten 1,9 %igen ASH/Methylenchlorid-Lösung wurden ohne weitere Reinigungsoperationen im Vakuum eingedampft. Der Rückstand wurde in derselben Gewichtsmenge Methanol gelöst und danach mit 20 %iger KOH/Methanol auf pH 8 bis 10 gebracht. Nach Abfiltrieren und Trocknen wurden 96,5 g Roh-ASK isoliert (Ausbeute: 98 %, bezogen auf 66 g eingesetztes ASH), das zu 83 % aus ASK bestand mit einem $K_2SO_4$-Gehalt von 8,8 % (bezogen auf ASK)

Beispiel 2

Rückführung der bei der Extraktion der organischen ASH-Phase mit Wasser anfallenden wässrige Phase in die Hydrolysestufe.

Für die Ringschlußreaktion wurde jeweils 1/10 der Menge der Einsatzprodukte wie in Beispiel 1 beschrieben eingesetzt. Die anfallende ASH/Methylenchlorid-Lösung (500 ml) wurde dann jeweils mit 50 ml Wasser 2 Stunden lang gerührt. Die hierbei anfallende Wasserphase wurde dann für die Hydrolyse des nächsten Versuchs eingesetzt.

Nach 10 Versuchen mit 9 Rückführungen der Wasserphase wurden durch KOH/Methanol-Fällung (siehe Beispiel 1) die in Tabelle 1 zusammengestellten ASK-Proben isoliert.

Tabelle 1

| Versuch Nr. | ASK (g) | Analyse ASK (%) | $K_2SO_4$ (%) |
|---|---|---|---|
| 1 | 13,8 | 99,9 | 0,3 |
| 2 | 15,7 | 99,5 | 0,4 |
| 3 | 16,0 | 99,5 | 0,5 |
| 4 | 16,4 | 99,7 | 0,3 |
| 5 | 16,2 | 99,9 | 0,2 |
| 6 | 15,9 | 100,0 | 0,2 |
| 7 | 16,5 | 99,8 | 0,3 |
| 8 | 16,2 | 99,6 | 0,4 |
| 9 | 16,3 | 99,6 | 0,5 |
| 10 | 16,0 | 99,9 | 0,2 |

Beispiel 3

Extraktive Neutralisation der ASH/Methylenchlorid-Phase mit 7 %iger Kalilauge.

5,0 l der nach Beispiel 1 hergestellten 1,9 %igen ASH/Methylenchlorid-Lösung wurden 2 Stunden lang mit 500 ml Wasser gerührt. Die organische Phase (112 g ASH enthaltend) wurde abgetrennt und mit 600 g 7 %iger Kalilauge 1,5 Stunden lang gerührt. Anschließend wurde die wässrige Phase abgetrennt. Von dieser Lösung wurden im Vakuum (60 mbar) 490 g Wasser abdestilliert. Nach Abkühlen des Rückstandes auf 0°C, Abfiltrieren und Trocknen wurden 115,7 g ASK isoliert.
Ausbeute: 84 % (bezogen auf 112 g ASH).

| Analyse: | ASK | $K_2SO_4$ |
|---|---|---|
| | 99,9% | 0,05% |

Nach weiterem Eindampfen der Kristallisationsmutterlauge wurden weitere 13,0 g ASK isoliert.
Ausbeute: 9% (bezogen auf 112 g ASH).

| Analyse: | ASK | $K_2SO_4$ |
|---|---|---|
| | 99,8% | 0,3% |

Beispiel 4

Extraktive Neutralisation der ASH/Methylenchlorid-Phase mit 30 %iger Kalilauge im Rührkolben.

5,0 l der nach Beispiel 1 hergestellten ASH/Methylenchlorid-Lösung wurden 2 Stunden lang mit 500 ml Wasser gerührt. Die organische Phase (= 112 g ASH) wurde abgetrennt und 0,5 Stunden lang mit 144,0 g 30 %iger KOH gerührt. Anschließend wurde das Reaktionsgemisch filtriert. Nach dem Trocknen wurden 112,8 g ASK erhalten.
Ausbeute: 81,5 % (bezogen auf 112 g ASH).

| Analyse: | ASK | $K_2SO_4$ |
|---|---|---|
| | 99,8% | 0,1% |

Beispiel 5

Extraktive Neutralisation der ASH/Methylenchlorid-Phase mit 30 %iger Kalilauge in einem Dünnfilmreaktor.

Die Apparatur bestand aus einem handelsüblichen Labordünnschichtverdampfer mit einer effektiven Länge von 22 cm und einer effektiven Fläche von 160 $cm^2$, der als Dünnfilmreaktor betrieben wurde. In diesen Reaktor wurden 2,5 l der wie in Beispiel 4 mit Wasser behandelten ASH/Methylenchlorid-Lösung und gleichzeitig 65,4 g 30 %ige KOH innerhalb von einer Stunde eingepumpt (Rotordrehzahl ca 800 UpM). Mittels einer Saugnutsche wurde aus dem den Reaktor verlassenden Reaktionsgemisch kontinuierlich das entstandene ASK abfiltirert. Nach Trocknen erhielt man 56,1 g ASK.
Ausbeute: 81 % (bezogen auf 56 g ASH).

| Analyse: | ASK | $K_2SO_4$ |
|---|---|---|
| | 99,7% | 0,3% |

Aus dem Totvolumen des Reaktors und den Volumenströmen der Einsatzprodukte errechnet sich eine mittlere Verweilzeit von 2,5 sec.

Beispiel 6

Abdestillieren des $CH_2Cl_2$ in einem Dünnschichtverdampfer unter Zusatz von Wasser.

Die Apparatur bestand aus einem handelsüblichen Labordünnschichtverdampfer mit einer effektiven Länge von 22 cm und einer effektiven Fläche von 160 $cm^2$. Bei einer Temperatur des Heizmantels von 115°C wurden 5,0 l/h einer wie in Beispiel 3 mit 500 ml Wasser behandelten ASH/$CH_2Cl_2$-Lösung ( 112 g ASH) und gleichzeitig 180 g Wasser/h in den Verdampfer fließen gelassen. Die aus dem Verdampfer ablaufende ca. 60 %ige ASH/Wasser-Lösung wurde nach Abkühlen auf Raumtemperatur mit 78,5 g 50 %iger Kalilauge unter Rühren neutralisiert. Nach Abkühlen auf 0°C wurden 110,7 g ASK isoliert.
Ausbeute: 80 % (bezogen auf 112 g ASH).

| Analyse: | ASK | $K_2SO_4$ |
|---|---|---|
| | 99,9% | 0,2% |

Nach weiterem Eindampfen der Kristallisations-Mutterlauge wurden nochmals 12,0 g ASK isoliert. Ausbeute: 9 % (bezogen auf 112 g ASH).

| Analyse: | ASK | $K_2SO_4$ |
|---|---|---|
| | 99,7% | 0,3% |

Beispiel 7

Abdestillieren des $CH_2Cl_2$ in einem Dünnschichtverdampfer unter Zusatz von Kalilauge.

In dieselbe Apparatur wie in Beispiel 6 wurden bei einer Temperatur des Heizmediums von 115°C 5,0 l/h der gleichen ASH/$CH_2Cl_2$-Lösung wie in Beispiel 6 und gleichzeitig 250 g 16 %ige Kalilauge eingespeist. Aus dem Verdampfer lief eine homogene wässrige ASK-Lösung ab (T = 105°C). Nach Abkühlen dieser Lösung auf 0°C wurde das auskristallisierte ASK abfiltriert und im Vakuum getrocknet.
Ausbeute: 127,2 g (92 %). (bezogen auf 112 g ASH).

| Analyse: | ASK | $K_2SO_4$ |
|---|---|---|
| | 99,9% | 0,1% |

**Patentansprüche**

1. Verfahren zur Herstellung der nicht-toxischen Salze des 6-Methyl-3,4-dihydro-1,2,3-oxathiazin-4-on-2,2-dioxids durch Ringschluß eines Acetoacetamid-Derivats und Neutralisation mit Basen, dadurch gekennzeichnet,
– daß man als Acetoacetamid-Derivat Acetoacetamid-N-sulfonsäure oder deren Salze – gelöst in einem mit Wasser nicht mischbaren inerten organischen Lösemittel – verwendet,
– daß man den Ringschluß durch die Einwirkung der mindestens äquimolaren Menge $SO_3$ – gegebenenfalls in gleicher Weise gelöst in einem mit Wasser nicht mischbaren inerten organischen Lösemittel oder auch in einem inerten anorganischen Lösemittel – durchführt,
– daß man im Falle des Einsatzes einer mehr als äquimolaren Menge $SO_3$ das nach der Ringschlußreaktion als $SO_3$-Addukt anfallende 6-Methyl-3,4-dihydro-1,2,3-oxathiazin-4-on-2,2-dioxid hydrolysiert,
– daß man die vorliegende oder bei der Hydrolyse sich absondernde organische Phase durch Extraktion mit Wasser oder 2 bis 20%iger wäßriger Schwefelsäure – vorzugsweise nur Wasser – im Volumenverhältnis der organischen Phase zur wäßrigen bzw. wäßrig-schwefelsauren Phase von (20–5):1 reinigt
– und daß man aus der so gereinigten organischen Phase die nicht-toxischen Salze des 6-Methyl-3,4-dihydro-1,2,3-oxathiazin-4-on-2,2-dioxids durch Neutralisation mit Basen gewinnt.

2. Verfahren nach Anspruch 1, dadurch gekennzeichnet, daß man Lösungen der Acetoacetamid-N-sulfonsäure oder von deren Salzen und von $SO_3$ in dem gleichen mit Wasser nicht mischbaren inerten organischen Lösemittel, vorzugsweise einem aliphatischen Chlorkohlenwasserstoff, insbesondere Methylenchlorid, verwendet.

3. Verfahren nach Anspruch 1 oder 2, dadurch gekennzeichnet, daß man den Ringschluß durch die Einwirkung einer mehr als äquimolaren Menge $SO_3$, in bezug auf Acetoacetamid-N-sulfonsäure oder von deren Salzen, durchführt.

4. Verfahren nach einem oder mehreren der Ansprüche 1 bis 3, dadurch gekennzeichnet, daß man die bei der Hydrolyse des 6-Methyl-3,4-dihydro-1,2,3-oxathiazin-4-on-2,2-dioxid-Addukts entstehende wäßrig-schwefelsaure Phase – zusammen mit gegebenenfalls ausgefallenem 6-Methyl-3,4-dihydro-1,2,3-oxathiazin-4-on-2,2-dioxid – noch mit einem mit Wasser nichtmischbaren inerten organischen Lösemittel – vorzugsweise dem gleichen, in dem auch die Ringschlußreaktion durchgeführt wurde, extrahiert und den organischen Extrakt mit der bei der Hydrolyse abgesonderten organischen Phase vereinigt.

5. Verfahren nach einem oder mehreren der Ansprüche 1 bis 4, dadurch gekennzeichnet, daß man das zur extraktiven Reinigung der organischen Phase verwendete Wasser oder die dazu auch verwendete verdünnte wäßrige Schwefelsäure wieder in die Hydrolyse des 6-Methyl-3,4-dihydro-1,2,3-oxathiazin-4-on-2,2-dioxid-Addukts zurückführt.

6. Verfahren nach einem oder mehreren der Ansprüche 1 bis 5, dadurch gekennzeichnet, daß man die Gewinnung der nichttoxischen Salze des 6-Methyl-3,4-dihydro-1,2,3-oxathiazin-4-on-2,2-dioxids aus der gereinigten organischen Phase mit der freien Dioxidverbindung durch Eindampfen der organischen Phase und Neutralisation des Rückstandes mit Basen, vorzugsweise Kaliumbasen, insbesondere mit KOH, durchführt.

7. Verfahren nach einem oder mehreren der Ansprüche 1 bis 5, dadurch gekennzeichnet, daß man die Neutralisation des 6-Methyl-3,4-dihydro-1,2,3-oxathiazin-4-on-2,2-dioxids durch intensiven Kontakt der diese Verbindung enthaltenden gereinigten organischen Phase mit einer wäßrigen Base, vorzugsweise einer wäßrigen Kaliumbase, insbesondere mit wäßriger KOH, durchführt.

8. Verfahren nach einem oder mehreren der Ansprüche 1 bis 5, dadurch gekennzeichnet, daß man zur Neutralisation des 6-Methyl-3,4-dihydro-1,2,3-oxathiazin-4-on-2,2-dioxids aus der diese Verbindung enthaltenden gereinigten organischen Phase das organische Lösungsmittel unter gleichzeitiger Zugabe

von Wasser verdampft und die wäßrige Lösung des 6-Methyl-3,4-dihydro-1,2,3-oxathiazin-4-on-2,2-dioxids dann mit einer Base, vorzugsweise einer Kaliumbase, insbesondere KOH, neutralisiert.

9. Verfahren nach einem oder mehreren der Ansprüche 1 bis 5, dadurch gekennzeichnet, daß man zur Neutralisation des 6-Methyl-3,4-dihydro-1,2,3-oxathiazin-4-on-2,2-dioxids aus der diese Verbindung enthaltenden gereinigten organischen Phase das organische Lösemittel unter gleichzeitiger Zugabe von wäßriger Base, vorzugsweise einer wäßrigen Kaliumbase, insbesondere von wäßriger KOH, verdampft.

10. Verfahren nach Anspruch 8 oder 9, dadurch gekennzeichnet, daß man das Verdampfen des organischen Lösemittels unter gleichzeitiger Zugabe von Wasser bzw. von wäßriger Base im Dünnschicht- oder Fallfilmverdampfer durchführt.

## Claims

1. A process for the preparation of the non-toxic salts of 6-methyl-3,4-dihydro-1,2,3-oxathiazin-4-one 2,2-dioxide by cyclizing an acetoacetamide derivative and neutralization with bases, which comprises

– using acetoacetamide-N-sulfonic acid or its salts dissolved in a water-immiscible, inert organic solvent as the acetoacetamide derivative,

– carrying out the cyclization by treatment with an at least equimolar amount of $SO_3$ – if appropriate similarly dissolved in a water-immiscible, inert organic solvent or in an inert inorganic solvent,

– in the event that the amount of $SO_3$ employed is more than equimolar, hydrolyzing the 6-methyl-3,4-dihydro-1,2,3-oxathiazin-4-one 2,2-dioxide obtained in the form of the $SO_3$-adduct after the cyclization reaction,

– purifying the organic phase which is present, or which separates out in the hydrolysis, by extraction with water or 2 to 20% aqueous sulfuric acid – preferably only water – in a volume ratio of the organic phase to the aqueous or aqueous sulfuric acid phase of (20–5):1,

– and isolating, by neutralization with bases, the non-toxic salts of the 6-methyl-3,4-dihydro-1,2,3-oxathiazin-4-one 2,2-dioxide from the organic phase thus purified.

2. The process as claimed in claim 1, wherein solutions of acetoacetamide-N-sulfonic acid or its salts and of $SO_3$ in the same water-immiscible, inert organic solvent, preferably an aliphatic chlorinated hydrocarbon and especially methylene chloride, are used.

3. The process as claimed in claim 1 or 2, wherein the cyclization is carried out by treatment with a more than equimolar amount of $SO_3$, relative to the acetoacetamide-N-sulfonic acid or its salts.

4. The process as claimed in any one or more of claims 1 to 3, wherein the aqueous sulfuric acid phase formed in the hydrolysis of the 6-methyl-3,4-dihydro-1,2,3-oxathiazin-4-one 2,2-dioxide adduct – together with 6-methyl-3,4-dihydro-1,2,3-oxathiazin-4-one 2,2-dioxide which may have been precipitated – is again extracted with a water-immiscible, inert organic solvent – preferably the same solvent in which the cyclization reaction was also carried out – and the organic extract is combined with the organic phase which separated out in the hydrolysis.

5. The process as claimed in any one or more of claims 1 to 4, wherein the water used for the purification by extraction of the organic phase, or the dilute aqueous sulfuric acid also used for this purpose, is recycled to the hydrolysis of the 6-methyl-3,4-dihydro-1,2,3-oxathiazin-4-one 2,2-dioxide adduct.

6. The process as claimed in any one or more of claims 1 to 5, wherein the isolation of the non-toxic salts of 6-methyl-3,4-dihydro-1,2,3-oxathiazin-4-one 2,2-dioxide from the purified organic phase containing the free dioxide compound is effected by evaporating the organic phase and neutralizing the residue with bases, preferably potassium bases and especially with KOH.

7. The process as claimed in any one or more of claims 1 to 5, wherein the neutralization of the 6-methyl-3,4-dihydro-1,2,3-oxathiazin-4-one 2,2-dioxide is carried out by effecting intensive contact between the purified organic phase containing this compound and an aqueous base, preferably an aqueous potassium base and especially aqueous KOH.

8. The process as claimed in any one or more of claims 1 to 5, wherein the 6-methyl-3,4-dihydro-1,2,3-oxathiazin-4-one 2,2-dioxide from the purified organic phase containing this compound is neutralized by evaporating off the organic solvent, with the simultaneous addition of water, and then neutralizing the aqueous solution of the 6-methyl-3,4-dihydro-1,2,3-oxathiazin-4-one 2,2-dioxide with a base, preferably a potassium base and especially KOH.

9. The process as claimed in any one or more of claims 1 to 5, wherein the 6-methyl-3,4-dihydro-1,2,3-oxathiazin-4-one 2,2-dioxide from the purified organic phase containing this compound is neutralized by evaporating off the organic solvent, with the simultaneous addition of an aqueous base, preferably an aqueous potassium base and especially aqueous KOH.

10. The process as claimed in claim 8 or 9, wherein the removal of the organic solvent by evaporation with the simultaneous addition of water or of an aqueous base is carried out in a thin film evaporator or a falling film evaporator.

## Revendications

1. Procédé de préparation de sels non-toxiques du 2,2-dioxyde de la 6-méthyl-3,4-dihydro-1,2,3-oxathiazin-4-one par cyclisation d'un dérivé d'acétoacétamide, puis neutralisation avec une base, procédé caractérisé en ce que:

– on emploie comme dérivé d'acétoacétamide l'acide acétoacétamide-N-sulfonique ou un sel de celui-ci, en solution dans un solvant organique inerte non-miscible à l'eau,

– on effectue la cyclisation en faisant agir une proportion au moins équimolaire de $SO_3$, éventuellement aussi en solution dans un solvant organique inerte non-miscible à l'eau ou dans un solvant minéral inerte,

– si l'on a utilisé une proportion de $SO_3$ supérieure à la proportion équimolaire, on hydrolyse le 6-méthyl-3,4-dihydro-1,2,3-oxathiazin-4-one-2,2-dioxyde qui a été obtenu, à la suite de la réaction de cyclisation, à l'état de produit d'addition de $SO_3$,

– on purifie la phase organique présente ou qui se sépare à l'hydrolyse par extraction avec de l'eau ou avec de l'acide sulfurique aqueux à une concentration de 2 à 20%, de préférence avec de l'eau seule, dans un rapport en volumes de la phase organique à la phase aqueuse ou d'acide sulfurique aqueux compris entre 20:1 et 5:1, et

– de la phase organique ainsi purifiée on obtient par neutralisation avec une base le sel non-toxique correspondant du 2,2-dioxyde de la 6-méthyl-3,4-dihydro-1,2,3-oxathiazin-4-one.

2. Procédé selon la revendication 1, caractérisé en ce que l'on utilise des solutions de l'acide acétoacétamide-N-sulfonique ou de sels de cet acide et de $SO_3$ dans le même solvant organique inerte non-miscible à l'eau, de préférence dans un hydrocarbure aliphatique chloré et notamment dans du chlorure de méthylène.

3. Procédé selon la revendication 1 ou 2, caractérisé en ce que l'on effectue la cyclisation avec une proportion de $SO_3$ supérieure à la proportion équimolaire par rapport à l'acide acétoacétamide-N-sulfonique ou à son sel.

4. Procédé selon l'une ou plusieurs des revendications 1 à 3, caractérisé en ce que l'on extrait encore la phase sulfurique aqueuse résultant de l'hydrolyse du produit d'addition du 6-méthyl-3,4-dihydro-1,2,3-oxathiazin-4-one-2,2-dioxyde, avec le 6-méthyl-3,4-dihydro-1,2,3-oxathiazin-4-one-2,2-dioxyde qui peut avoir éventuellement précipité, par un solvant organique inerte non-miscible à l'eau, de préférence le même que celui dans lequel la cyclisation a été réalisée, et on réunit l'extrait organique à la phase organique séparée dans l'hydrolyse.

5. Procédé selon l'une ou plusieurs des revendications 1 à 4, caractérisé en ce que l'on renvoie à l'hydrolyse du produit d'addition du 6-méthyl-3,4-dihydro-1,2,3-oxathiazin-4-one-2,2-dioxyde l'eau ou l'acide sulfurique aqueux dilué ayant servi à la purification par extraction de la phase organique.

6. Procédé selon l'une ou plusieurs des revendications 1 à 5, caractérisé en ce que l'on obtient les sels non-toxiques du 6-méthyl-3,4-dihydro-1,2,3-oxathiazin-4-one-2,2-dioxyde de la phase organique purifiée du dioxyde libre par évaporation de cette phase organique et neutralisation de la matière restante avec une base, de préférence une base de potassium et notamment d'hydroxyde de potassium.

7. Procédé selon l'une ou plusieurs des revendications 1 à 5, caractérisé en ce que l'on neutralise le 6-méthyl-3,4-dihydro-1,2,3-oxathiazin-4-one-2,2-dioxyde par un contact intense de la phase organique purifiée contenant ce composé avec une base aqueuse, de préférence une base de potassium aqueuse et notamment une solution aqueuse d'hydroxyde de potassium.

8. Procédé selon l'une ou plusieurs des revendications 1 à 5, caractérisé en ce que pour neutraliser le 6-méthyl-3,4-dihydro-1,2,3-oxathiazin-4-one-2,2-dioxyde de la phase organique purifiée contenant ce composé, on évapore le solvant organique tout en ajoutant en même temps de l'eau, puis on neutralise la solution aqueuse du 2,2-dioxyde avec une base, de préférence une base de potassium et en particulier de l'hydroxyde de potassium.

9. Procédé selon l'une ou plusieurs des revendications 1 à 5, caractérisé en ce que pour neutraliser le 6-méthyl-3,4-dihydro-1,2,3-oxathiazin-4-one-2,2-dioxyde de la phase organique purifiée contenant ce composé, on évapore le solvant organique tout en ajoutant en même temps une base aqueuse, de préférence une base de potassium aqueuse et en particulier de l'hydroxyde de potassium aqueux.

10. Procédé selon la revendication 8 ou 9, caractérisé en ce que l'on effectue l'évaporation du solvant organique, avec addition simultanée d'eau ou d'une base aqueuse, dans un évaporateur à couche mince ou à pellicule tombante.